Europäisches Patentamt

European Patent Office

Office européen des brevets

(19)

(11) Numéro de publication: **0 394 149**
**A1**

## (12) DEMANDE DE BREVET EUROPEEN

(21) Numéro de dépôt: **90420189.4**

(22) Date de dépôt: **13.04.90**

(51) Int. Cl.⁵: **A61F 13/08**

(30) Priorité: **18.04.89 FR 8905501**

(43) Date de publication de la demande:
**24.10.90 Bulletin 90/43**

(84) Etats contractants désignés:
**AT BE CH DE DK ES GB GR IT LI LU NL SE**

(71) Demandeur: **GANZONI & CIE (Société Anonyme)**
**13 Rue de Village Neuf**
**F-68304 Saint Louis(FR)**

(72) Inventeur: **Ganzoni,Stéfan**
**Kirschbaumweg 46**
**CH-4203 Bottminjen(CH)**

(74) Mandataire: **Dupuis, François**
**Cabinet Charras, 3 Place de l'Hôtel-de-Ville, BP 203**
**F-42005 St. Etienne Cédex 1(FR)**

(54) Bas à usage médical et produit dérivé.

(57) Le bas est remarquable en ce qu'il présente sensiblement au-dessus de la partie entourant la cheville et la région malléolaire dans son plan transversal périphérique sur tout ou partie du bas à l'endroit considéré, un moyen (6) de visualisation et de référence, ce moyen (6) étant combiné avec une partie de référence (7) complémentaire formée sur le bas côté pied, la distance de séparation du moyen (6) et de la partie de référence définissant la taille du bas correspondant.

FIG.1

L'invention a pour objet un bas à usage médical et produit dérivé.

L'invention se rattache au secteur technique des articles vestimentaires du type bas, collants et produits dérivés, en particulier à usage médical.

Il est d'usage courant de réaliser des bas selon différentes tailles pour s'adapter à la morphologie des patients. Les tailles sont définies par des mesures de tour de cheville, tour de cuisse et sont généralement indiquées sur le boîtier de conditionnement des articles. Les tailles identifiées par les références T1, T2, T3... ou les lettres A, B, C, ... ou autres définissent en fait des fourchettes ou des tolérances de dimensions pour éviter de multiplier de manière inappropriée et inconsidérée les différents types de bas. En effet, on doit tenir compte d'impératifs commerciaux et de gestion de stocks et éviter ou limiter les coûts en résultant. Généralement, les bas sont impressionnés avec la marque du fabricant et la référence générique de la taille mais sans information détaillée. En outre, l'utilisateur a connaissance de la taille qui lui est nécessaire dans sa notion d'ensemble et non la mesure exacte.

Dans certains cas, d'autres personnes que l'utilisateur lui-même ont à choisir et sélectionner le bas à porter. C'est par exemple le cas dans les milieux hospitaliers pour le port de bas ou collants médicaux. Ceux-ci sont généralement sortis de leur boîtier et seule la référence générique de la taille T1 - T2 ... apparaît. Les infirmiers et autres agents qui doivent sélectionner les bas pour les enfiler sur les jambes des patients n'ont eux aucune idée de la taille réelle du tour de cheville ou du tour de cuisse du patient considéré. De plus, ils n'ont aucun moyen particulier de mesure du type mètre enroulable ou autre. En pratique, l'infirmier est amené à apprécier lui-même la taille appropriée du bas à défaut de pouvoir obtenir une information précise de son patient. Ainsi, il arrive souvent que les bas posés ne correspondent pas à la taille réelle et nécessaire. L'effet thérapeutique recherché avec des bas ou collants médicaux n'est donc pas atteint soit par une insuffisance de contention, ou au contraire une force de pression trop forte.

Il y a lieu également de signaler que le choix des tailles de bas en général peut varier en fonction de l'âge du patient.

En outre, les différentes tailles proposées par les fabicants ne correspondent pas toutes à des mesures identiques de sorte que le choix d'un type de bas résulte souvent de conditions empiriques.

Le but recherché selon l'invention était donc de concevoir un bas et produits dérivés d'aspect nouveau permettant de définir la taille la plus appropriée aux jambes d'un patient.

Un autre but selon l'invention était de proposer un bas et produits dérivés répondant aux différents problèmes posés sans pour autant entraîner des surcoûts de fabrication.

Un autre but selon l'invention était de proposer un moyen rapide d'identification de la taille d'un bas et produits dérivés la plus appropriée.

Ces buts et d'autres encore ressortiront bien de la suite de la description.

Selon une première caractéristique de l'invention, le bas ou produits dérivés est remarquable en ce qu'il présente sensiblement au-dessus de la partie entourant la cheville et la région malléolaire dans son plan transversal périphérique sur tout ou partie du bas à l'endroit considéré, un moyen de visualisation et de référence, ce moyen étant combiné avec une partie de référence complémentaire formée sur le bas côté pied, la distance de séparation du moyen et de la partie de référence définissant la taille du bas correspondant.

Selon une autre caractéristique, le moyen est établi par une ou des lignes ou liserés de couleur ou autres signes définissant une zône ou bande d'une certaine longueur correspondant à une plage de tolérances dimensionnelles en égard de la partie de référence.

Ces caractéristiques et d'autres encore ressortiront bien de la suite de la description.

Pour fixer l'objet de l'invention, d'une manière non limitative illustrée aux figures des dessins où :

La figure 1 est une vue d'un bas sans pointe selon l'invention.

La figure 2 est une vue d'un bas avec pointe selon l'invention.

La figure 3 est une vue d'un bas sans pointe selon une variante de réalisation.

La figure 4 est une vue illustrant une partie de bas enroulé autour de la cheville en vue de déterminer, vérifier et comparer la taille du tour de cheville en égard de la taille du bas proposé.

La figure 5 est une vue en variante d'un produit dérivé tel que collant réalisé et agencé selon l'invention.

Afin de rendre plus concret l'objet de l'invention, on le décrit maintenant d'une manière non limitative en se référant aux exemples de réalisation des figures des dessins.

Le bas (1) est considéré dans son sens générique et peut être tissé ou tricoté de toutes manières et techniques appropriées avec tous types de mailles et présenter les caractéristiques désirées par chaque fabricant. Il peut comprendre des revers (2), sous-revers (3), des élément de renfort du talon (4) et à la pointe (5) si celle-ci est définie. Le bas et produits dérivés similaires peut être réalisé pour entourer tout ou partie du pied et/ou la jambe, se fixer sur la jambe ou le genou, ou se prolonger au-dessus du genou et se fixer sur la cuisse. Il peut également être réalisé sous la forme d'un collant.

L'invention consiste à disposer sur le bas sensiblement au-dessus de la partie entourant la cheville et des régions malléolaires dans un plan transversal périphérique sur tout ou partie du pourtour du bas à l'endroit considéré, un moyen (6) de visualisation et de référence. Ce moyen (6) est tissé, tricoté avec le bas, en donnant toutefois un aspect visuel différent facile à identifier. Ce moyen peut être établi par une ou des lignes ou liserés superposés de même couleur, ou par une ligne en zig-zag continue ou par tous autres signes d'identification. Ces derniers peuvent être établis sur tout ou partie de la périphérie du bas à l'endroit du tour de cheville. Avantageusement, ce moyen définissant une zône ou bande est établi avec une certaine largeur pour définir une plage de tolérance de dimensions ainsi qu'il sera précisé pa la suite. Ce moyen (6) est combiné avec une partie de référence complémentaire (7) formée sur le bas du côté pied. Si le bas ne présente pas de pointe, la partie de référence sera établie par la ligne de bordure extérieure (8) du bas ou par une ligne matérialisée (8.1) adjacente. Il peut être prévu à l'extrémité de celui-ci une bordure ou bande de contention ainsi qu'illustré aux dessins. Dans l'hypothèse où le bas présente en bout une pointe, la partie de référence est matérialisée par un signe (9), liserés ou similaires, formé également lors de la fabrication du bas. Ce signe est disposé transversalement sur la périphérie de la partie pied du bas et est établi par une ligne colorée ou liseré de manière différente de celle du bas.

Ainsi, on définit entre la partie de référence (7) qui est soit la bordure (8) ou bande de contention à l'extrémité du bas, soit la ligne intermédiaire (8.1), soit le signe (9), et le moyen (6) défini par des lignes (6.1 - 6.2) d'extrémités côté pied et côté jambe, des distances ou cotes déterminées (L1 - L2) variables sensiblement en fonction de la largeur dudit moyen. On obtient ainsi deux limites (a) et (b) avec une plage de tolérance (c). Les dimensions définies sont établies pour correspondre à des tours de chevilles différents mais dans une limite particulière pour correspondre à une taille donnée. A titre d'exemple non limitatif, la distance (a) correspond à un tour de cheville de 19 cm, et la distance (b) à un tour de cheville de 23 cm, l'ensemble définissant la taille (1), avec une plage de tolérance de 4 centimètres.

Pour permettre une mesure précise, les distances (a) et (b) sont déterminées en prenant référence en joignant les points milieux (A) de la partie de référence (7) du bas posé à plat et non enfilé et (B) et (C) des lignes d'extrémité du moyen (6). On doit en effet tenir compte que les arcs d'enroulement (d) et (e) reliant les extrémités supérieure côté cou de pied et inférieur côté talon de la partie de référence (7) et du moyen (6) sont différents pour tenir compte naturellement de la conformation du bas adapté à la morphologie du pied.

On peut néanmoins prévoir en variante que les distances de référence (a) et (b) définissant les limites extrêmes des mesures et donc d'une taille déterminée, soient prises soit à partir des extrémités supérieures (A', B', C') côté cou de pied du bas en position à plat et non enfilé, soit à partir des extrémités inférieures (A'', B'', C'') côté talon du bas en position à plat et non enfilé, ainsi qu'illustrée aux dessins.

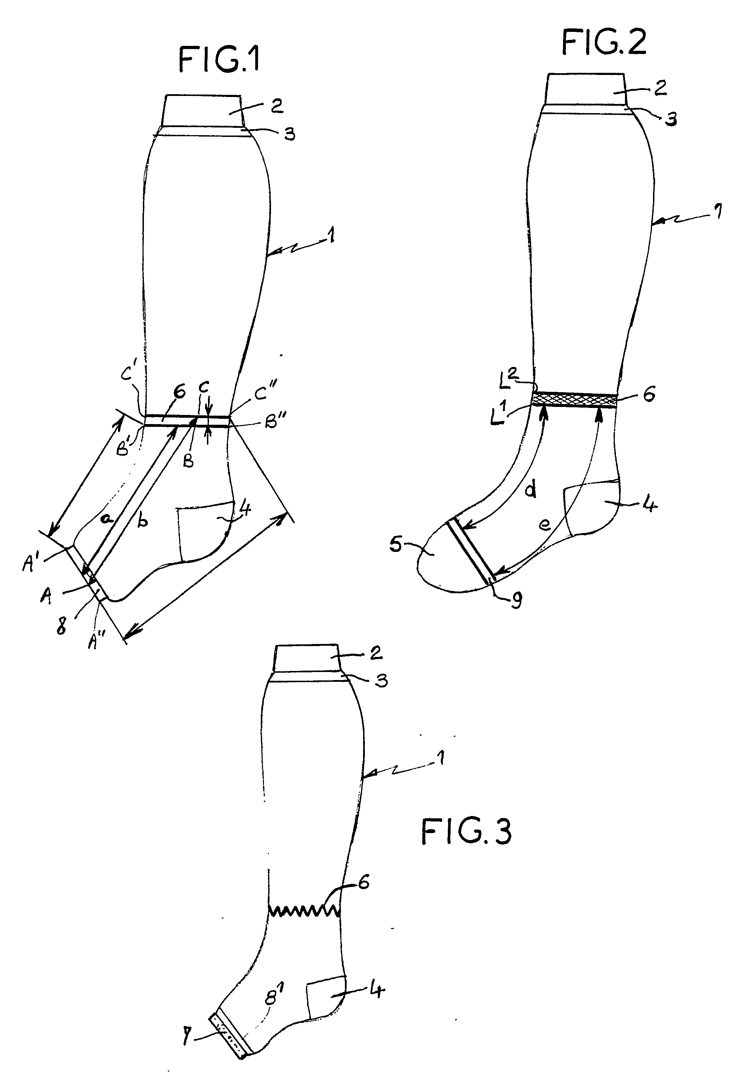

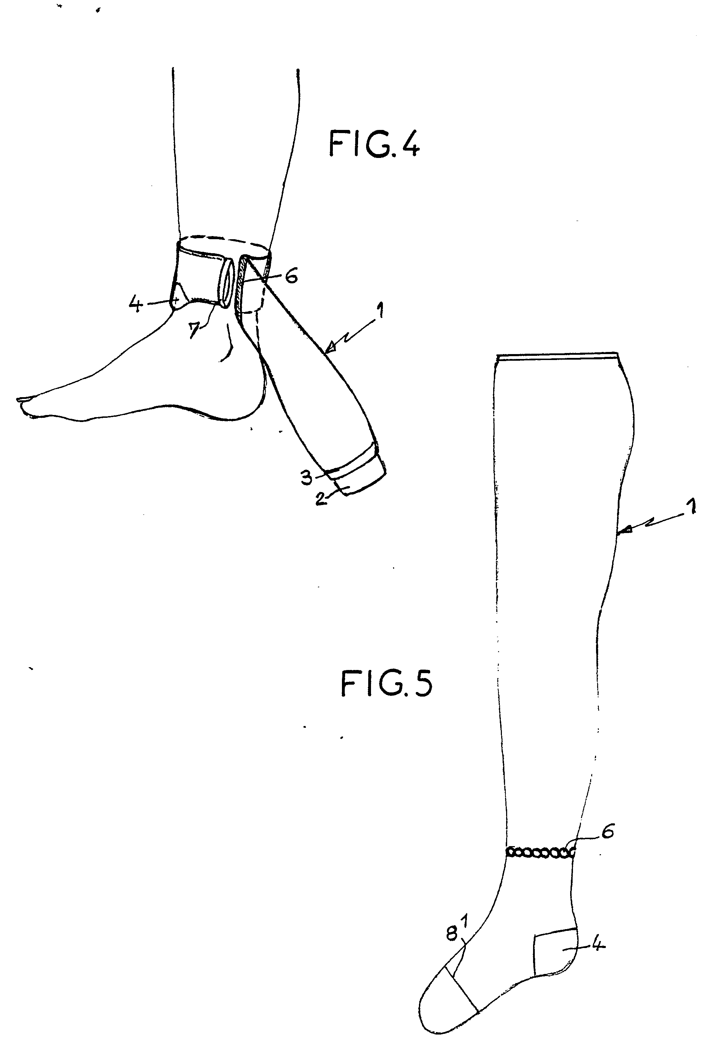

Afin de déterminer si le bas choisi correspond à la taille de son utilisateur potentiel, on procède de la manière suivante dans une première mise en oeuvre, en faisant référence à la figure 4 des dessins. On saisit le bas que l'on positionne par sa partie pied autour de la cheville sensiblement au-dessus de la région malléolaire. La partie de référence (7) est sensiblement verticale dans le sens de la jambe, tandis que la partie talon du bas se trouve orientée côté pied ; l'opérateur procède alors à un enroulement du bas autour de la cheville sans exercer de force de tension afin de ne pas déformer élastiquement le bas. Si après un enroulement effectué le moyen (6) de visualisation se situe en regard de la partie de référence (7) dans la plage (c) de tolérance préétablie, le bas considéré est apte à être enfilé dans de bonnes conditions sur la jambe du patient. Si, par contre, le moyen (6) se situe au-delà ou en-deça de la partie de référence (7), la taille présumée du bas est inappropriée au patient.

Le procédé de vérification est donc extrêmement rapide, pratique à effectuer et ne procure aucune gêne pour le patient ou utilisateur. En outre, indépendamment de la fonction recherchée, le moyen (6) confère une esthétique nouvelle aux bas précités ou produits dérivés. Le talon du bas étant orienté vers la partie pied lors de la mesure, il ne peut gêner celle-ci.

On peut toutefois procéder à la vérification en positionnant la partie du bas enveloppant la cheville de manière différente, le talon étant alors orienté côté jambe.

L'invention trouve de nombreux avantages. On souligne la simplicité de réalisation et de confection du bas, collant à usage médical.

**Revendications**

-1- Bas à usage médical et produits dérivés du type entourant au moins tout ou partie du pied puis la jambe et se fixant ou se prolongeant sur la cuisse d'un utilisateur en constituant un collant, caractérisé en ce que le bas présente sensiblement au-dessus de la partie entourant la cheville et la région malléolaire dans son plan transversal périphérique sur tout ou partie du bas à l'endroit considéré, un moyen (6) de visualisation et de référence, ce moyen (6) étant combiné avec une partie de référence (7) complémentaire formée sur le bas côté pied, la distance de séparation du moyen (6) et de la partie de référence définissant la taille du bas correspondant.

-2- Bas selon la revendication 1, caractérisé en ce que le moyen (6) et la partie de référence (7) sont réalisés lors de la fabrication du bas par tissage ou tricotage en conférant un aspect visuel distinct des autres parties du bas.

-3- Bas selon l'une quelconque des revendications 1 et 2, caractérisé en ce que le moyen (6) est établi par une ou des lignes ou liserés de couleur ou autres signes définissant une zône ou bande d'une certaine longueur correspondant à une plage de tolérances dimensionnelles en égard de la partie de référence (7).

-4- Bas selon l'une quelconque des revendications 1, 2 et 3 dans son application à un bas sans pointe, caractérisé en ce que la partie de référence (7) est définie par la ligne de bordure extrême (8) ou bande de contention du bas notamment le pied.

-5- Bas selon l'une quelconque des revendications 1, 2 et 3 dans son application à un bas sans pointe, caractérisé en ce que la partie de référence (7) est définie par une ligne matérialisée (8.1) adjacente à la bordure extrême ou bande de contention du bas entourant le pied.

-6- Bas selon l'une quelconque des revendications 1, 2 et 3 dans son application à un bas avec pointe, caractérisé en ce que la partie de référence (7) est définie par un signe (9), liserés ou similaires disposés transversalement sur la périphérie de la partie pied du bas.

-7- Bas selon l'une quelconque des revendications 4, 5 et 6, caractérisé en ce qu'une distance (a) est définie entre une ligne (L1) côté pied du moyen (6) et la bordure (8), par la bande intermédiaire (8.1) ou le signe (9) côté pied, et une distance (b) est définie entre une ligne (L2) côté jambe du moyn (6) et la bordure (8) ou la bande intermédiaire (8.1) ou le signe (9), une plage de tolérance (C) correspondant à la différence des distances (a - b) et correspondant à la longueur du moyen (6).

-8- Procédé de mesure et de sélection d'un bas en fonction de la taille d'un utilisateur selon l'une quelconque des revendications 1 à 7, caractérisé en ce que la mesure de référence est établie à partir des points milieux (A) de la partie (7) du bas posé à plat et non enfilé et des points milieux (B) et (C) des lignes (L1 - L2) côté pied côté jambe définissant la longueur du moyen (6).

-9- Procédé de mesure et de sélection d'un bas en fonction de la taille d'un utilisateur selon l'une quelconque des revendications 1 à 7, caractérisé en ce que la mesure de référence est établie à partir des extrémités supérieures (A', B', C') côté cou de pied du bas de la partie de référence (7) et des lignes (L1 - L2) côté pied côté jambe définissant la largeur du moyen (6).

-10- Procédé de mesure et de sélection d'un bas en fonction de la taille d'un utilisateur selon l'un quelconque des revendications 1 à 7, caractérisé en ce que la mesure de référence est établie à partir des extrémités inférieures (A", B", C") côté talon du bas de la partie de référence (7) et des lignes (L1 - L2) côté pied côté jambe définissant la largeur du moyen (6).

-11- Procédé de mesure selon l'une quelconque des revendications 8, 9 et 10, caractérisé en ce que l'on positionne le bas par sa partie pied autour de la cheville sensiblement au-dessus de la région malléolaire, la partie de référence (7) étant sensiblement dans le sens de la jambe, tandis que la partie talon du bas se trouve orientée côté pied ; on procède à un enroulement du bas autour de la cheville sans exercer de force de tension ; on compare après enroulement la position du moyen (6) par rapport à la partie de référence (7) pour vérifier si celle-ci se situe en regard de la plage (2) de tolérance préétablie afin de vérifier si la taille du bas contrôlé correspond à la taille réelle de la jambe de l'utilisateur, puis on utilise le bas choisi ou on en sélectionne un autre.

-12- Procédé de mesure selon l'une quelconque des revendications 8, 9 et 10, caractérisé en ce que l'on positionne le bas par sa partie pied autour de la cheville sensiblement au-dessus de la région malléolaire, la partie de référence (7) étant sensiblement dans le sens de la jambe tandis que la position talon se trouve orientée vers le haut de la jambe ; on procède à un enroulement du bas autour de la cheville sans exercer de force de tension ; on compare après enroulement la position du moyen (6) par rapport à la partie de référence (7) pour vérifier si celle-ci se situe en regard de la plage (2) de tolérance préétablie afin de vérifier si la taille du bas contrôlé correspond à la taille réelle de la jambe de l'utilisateur, puis on utilise le bas choisi ou on en sélectionne un autre.

EP 0 394 149 A1

FIG.1

FIG.2

FIG.3

FIG.4

6

4

7

1

3

2

FIG.5

1

1

6

8

4

Office européen
des brevets

**RAPPORT DE RECHERCHE EUROPEENNE**

Numero de la demande

EP 90 42 0189

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 502 301  (R.T. SWALLOW et al.) * revendications 1-3; figures 1,2 * --- | 1 | A 61 F   13/08 |
| A | US-A-2 816 361  (C. JOBST) * revendication 1 * --- | 1,8 | |
| A | US-A-3 217 336  (S.J. WIKLER) * figure 8 * ----- | 1 | |

**DOMAINES TECHNIQUES RECHERCHES (Int. Cl.5)**

A 61 F
A 41 B

Le présent rapport a été établi pour toutes les revendications

| Lieu de la recherche | Date d'achèvement de la recherche | Examinateur |
|---|---|---|
| BERLIN | 21-06-1990 | KANAL P K |

EPO FORM 1503 03.82 (P0402)